# EUROPEAN PATENT APPLICATION

(11) **EP 3 671 150 A1**
(43) Date of publication of application: **24.06.2020**
(21) Application number: 19216385.5
(22) Date of filing: 16.12.2019
(51) Int. Cl.: G01K 3/04, G01K 11/12

(54) **DEVICE FOR A PRODUCT TEMPERATURE VARIATION DETECTION BELOW A THRESHOLD VALUE**

(30) Priority: 17.12.2018 IT 201800011174
(71) Applicant: Bonomi, Renato, 36100 Vicenza (IT)
(72) Inventor: Bonomi, Renato, 36100 Vicenza (IT)
(74) Representative: Tiburzi, Andrea

(57) **Abstract**

The present invention relates to a device for monitoring a temperature variation undergone by a product, which detects a drop in temperature below a predetermined temperature threshold (T_{cs}), comprising: a sealed casing (I) which defines a containment space (V), and a mixture contained, or containable, in said containment compartment (V) which comprises a liquid phase (S) and a solid (D) comprising metal particles having an average nanometric size comprised between 1 and 300 nm and a coating layer (R) of said particles.

This coating (R) comprises an organic material and is configured in such a way that, in a configuration of use of the device at a first temperature (T₁) greater than said threshold temperature (T_{cs}), it allows the maintenance of the solid (D) in solution in said liquid phase (S), in which the particles are separated from each other, while at a crystallization temperature (T₂) of said liquid phase (S), being said temperature (T₂) equal to or lower than said threshold temperature (T_{cs}), the coating layer (R) separates from the metal particles allowing an aggregation of the metal particles. The mixture undergoes an irreversible loss of optical properties, allowing the detection of undesired temperature variation.

## Description

The present invention is in the field of sensors for detecting temperature variations, and in particular refers to a sensor that detects temperature drops below a predetermined threshold.

The definition of the temperature profile over time of a product is currently difficult to implement. Especially in the pharmaceutical and food sectors, there are products that deteriorate when subjected to an excessive lowering of the temperature, even if subsequently brought back to an ideal temperature.

This variation in the temperature of a product often leads to its alteration, which is not always visible with the naked eye. This often leads to fraud or concealment of anomalies in a product or part of it, as well as obvious consequences of toxicity for those who use it.

In particular, the detection of low temperatures makes it possible to establish whether a product, during transport or storage, has been stored at lower temperatures than permitted. Conventional methods for temperature monitoring are electronic or mechanical.

For example, digital thermometers can monitor temperature and record its progresses over time. In the last twenty years, mechanical devices have also been invented, for example described in documents US8028533B2 and US4191125, which include two substances separated by a septum; due to the freezing of one of the two substances, and its consequent dilatation, the septum breaks and the substances come into contact with each other. Their mixing causes irreversible color variation.

The commercial success of these devices has not been relevant due to their fragility, their high cost and not small size; they can also be easily removed and manipulated.

The patent n. RU2585464C1 describes a device for detecting thawing, based on the use of a carotenoid protein which, irradiated by a source with a wavelength of 450 ± 40 nm at a given intensity, takes on a red color, and is then frozen.

Following to a possible thawing, the color becomes orange, even in the case of a subsequent refreezing, but it may turn red after a further irradiation with the light at the same wavelength and at the same intensity.

Naturally, since these sensors serve to prevent tampering by third parties and to signal possible frauds, the possibility that this substance, subjected to such irradiation, returns to its original color, makes this system susceptible to alterations.

Therefore, remains the need to have a device for detecting the lowering of the temperature in a product below a threshold that is reliable over time and irreversible.

The main purpose of the invention is to realize a device for detecting the lowering of the temperature in a product below a certain threshold, which is therefore not able to be tampered by third parties.

Another aim of the invention is to obtain a device of the type mentioned which is easy and cheap to make and apply.

Furthermore, an aim of the invention is to obtain a device which is simple and immediate to be interpreted by the end user, so as to avoid misunderstandings or misinterpretations.

Furthermore, the invention aims to increase the compliance of a possible drug in patients having difficulties.

It is therefore object of the invention a device for monitoring a temperature variation to which a product has been subjected, which detects a lowering of the temperature below a predetermined threshold temperature.

This device comprises a sealed casing, which defines a containment compartment, and a mixture contained, or which can be contained, in the containment compartment.

The mixture comprises a liquid phase and a solid dissolved therein: the solid comprises metallic particles having a nanometric average dimension of between 1 and 300 nm and a coating layer of said particles.

The coating comprises organic molecules, and is configured in such a way that, in a configuration of use of the device at a first temperature, higher than the threshold temperature, the metallic particles of the solid in the solution can be maintained separated.

Instead, at a crystallization temperature of the liquid phase, being it equal to or lower than the threshold temperature, the coating separates from the metallic nanoparticles, causing an aggregation of the metal nanoparticles. Since this aggregation is irreversible, even if the temperature is brought again above the threshold value, the optical properties of the mixture in this configuration are different from those of the mixture containing the nanoparticles separated from each other, and therefore allows to highlight a temperature variation undergone by the device itself.

In a preferred embodiment, the invention provides that the coating comprises organic molecules arranged in a monolayer.

Furthermore, according to the invention, the liquid phase can comprise at least one of the following solvents: water, alcohols, ethers, hydrocarbons, esters, amides, sulfoxides, aldehydes, ketones, amines.

In this case, still according to the invention, the liquid phase can comprise at least one of: water, ethanol, ethylene glycol, methanol, propanol, butanol, propylamine, butylamine, methyl-terbutyl-ether (MTBE), dimethylsulfoxide (DMSO), methyl -ethyl ketone (MEK), dimethylformamide (DMF), acetone, acetonitrile, toluene, cyclohexane, hexane.

Furthermore, according to the invention, the coating can comprise at least one binding group selected from one of the following: thiols, alkylsulphides, disulfides, thioacids, thioesters, phosphines, amines, carboxylates, citrates, ascorbates, halides, ammonium salts, surfactants.

Furthermore, the coating can comprise at least one functional group selected from one of the following: phosphate, phosphonate, alcohols or glycols, amines, ammonium, ethers or polyethers, mono-oligo- or poly-saccharides, peptides, sulfite, sulfate, hydrocarbons, sulfonate and carboxylate.

Finally, another object of the invention is the use of a mixture for a device for monitoring a temperature variation compared to a threshold temperature undergone by a product, wherein the mixture comprises a liquid phase and a dissolved solid therein: this solid comprises metallic particles having an average nanometric size of between 1 and 300 nm and a coating layer of the particles.

This coating layer comprises an organic material and is configured in such a way that, in a configuration of use of the device at a first temperature, higher than the threshold temperature, the metallic particles of the solid can be maintained in solution in the liquid phase, in which the nanoparticles are separated; at a crystallization temperature of the liquid phase, the second temperature being equal to or lower than the threshold temperature, the coating layer separates from the metallic nanoparticles, allowing an aggregation of the metallic nanoparticles of the solid.

The strategy proposed in this invention allows in an advantageous and immediate way to detect with the naked eye whether, in the thermal history, the temperature has undergone variations below a certain threshold, with no need of electronic and/or mechanical devices.

In detail, the invention is based on the dispersion of nanoparticles that show plasmonic absorption properties in a suitable solvent; the mixture thus obtained is also called "colloidal solution" or "colloid".

These systems are now very well studied and understood and, for nanoparticle diameters between 1 and 300 nm, they show strong absorptions in the visible light spectrum, and they have a characteristic color which depends on the type of nanoparticle. However, the dispersion of nanoparticles in the solvent is only possible after the functionalization of their surface with proper chemical species (also called "passivating"), according to the well-known phenomenon of self-assembly (self-assembly). In particular, the nanoparticles having plasmonic properties are commonly stabilized by coating with a monolayer of organic molecules.

The stability of the nanoparticle-passivating complex in a given solvent depends on the nature of the organic monolayer and on the size of the nanoparticles. Here in particular, a monolayer means that the passivating forms a single layer onto the surface of the nanoparticle, and not more layers.

If the solvent were frozen, the bond between the surface of the particles and the passivating would break permanently and irreversibly, causing the aggregation of the nanoparticles themselves which, therefore, irreversibly aggregate precipitating.

The aggregation of the nanoparticles inhibits their optical behavior, and therefore that of the solution, depriving it of the characteristic color it had before the aggregation of the nanoparticles.

Passivated gold nanoparticles are a typical and easy to make example: in fact, they can be prepared in many ways, one of which consists in dissolving a salt containing Au (III) ions in water together with a binder and a reductant, for example citrate, and bringing the solution to a temperature of around 80°C. Citrate, following the increase of temperature, reduces the ion to metallic Au and also keeps the metal particles in suspension by binding to their surface; the result is a solution with a magenta-red coloration, which will be maintained until the solvent freezes.

Further features and advantages of the device for detecting the temperature variation undergone by a product below a certain threshold, according to the present invention, will become more evident from the description of an exemplary and favorite but not limiting embodiment and from the attached drawings, in which:

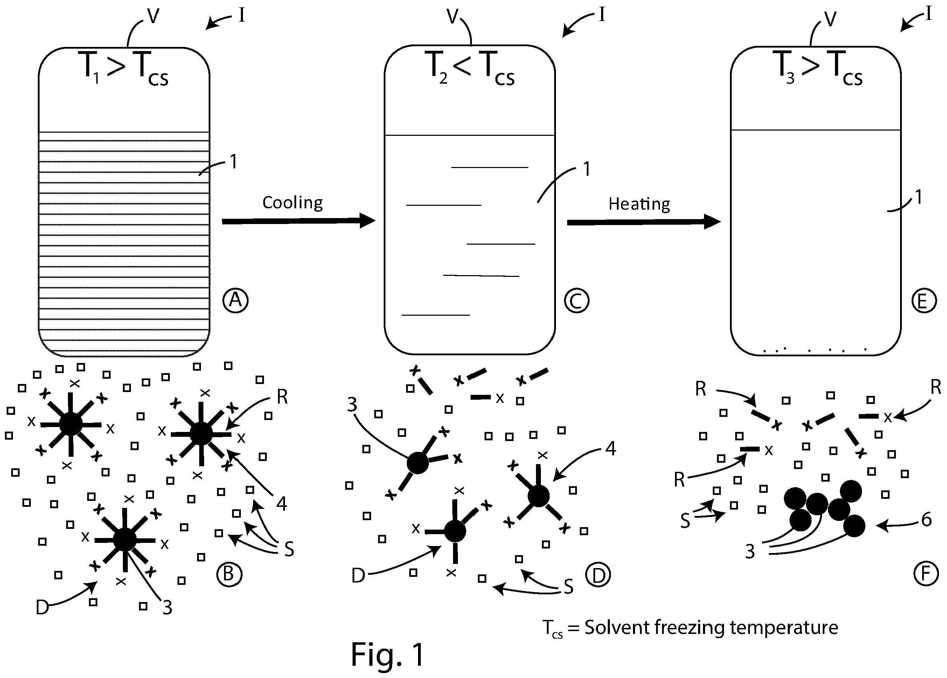

- figure 1 shows
- in A a schematic representation of a preferred embodiment of the device of the invention, in a first step;
- in B a schematic representation of a detail of the particles in suspension inside the device of Figure 1A;
- in C a schematic representation of a favorite embodiment of the device of the invention, in a second phase;
- in D a schematic representation of a detail of the particles inside the device of Figure 1C;
- in E a schematic representation of a favorite embodiment of the device of the invention, in a third phase;
- in F a schematic representation of a detail of the precipitated particles inside the device of Figure 1E;
- figure 2 shows a graph of the relative absorbance to the spectrum of the visible light of the mixture within the device of figures 1A and 1E;
- Figure 3 shows a representative diagram of the nature of the molecules used for stabilizing the particles of Figure 1B and some examples of bonding groups.

The invention consists of a system for detecting a change in temperature below a threshold, even when this change has subsequently been reversed. The system conceived makes it possible to detect whether the temperature value falls below a predetermined value. This value can be modified ad hoc, depending on the nature of the various components that make up the system; in other words, it can be modified according to the solvent (or liquid phase) S, and of the complex created by the metal nanoparticles and the coating R, hereinafter also referred to as the solid phase D.

The idea is based on the phenomenon of aggregation of metallic nanoparticles, following the freezing of the solvent in which they are dispersed.

The phenomenon is irreversible, due to the breakdown of the structure of the nanoparticle and of the subsequent precipitation of aggregates no longer dispersible in the solvent. Self-assembly is a molecular phenomenon through which a complex molecular system is spontaneously formed (such as in the case of human cells, proteins, viruses, etc.). In the present case, when nanoparticles are produced, the passivating agent self-assembles, attaching to the surface of the nanoparticle itself.

However, at its freezing temperature the solvent, crystallizing, destabilizes the organic monolayer causing its detachment from the surface of the nanoparticles; it is therefore a physical solicitation that induces the breaking of the nanoparticle-covering complex.

These organic molecules give these nanoparticle systems excellent stability. In fact, these coated nanoparticles can be produced and maintained at temperatures between 0 and 50 °C for long periods, they can be exposed to sunlight, dried and dissolved again in a new solvent.

The stability of the nanoparticle systems depends on the structure of the organic molecule and in particular on the strength of the surface-cover bond. In particular, in a preferred embodiment of the invention, it is possible to confine a variable quantity of mixture in a containment space, for example a sealed casing, to be applied onto a product whose thermal history is to be checked.

A system designed in this way can be used in the medical, pharmaceutical, food, agricultural, construction, and others sectors in order to trace the thermal history of a potentially degradable product.

One of the most interesting properties of nanoparticles is their absorption in the visible region, called plasmonic absorption, due to the electronic properties of the nucleus, or nucleus, (generally metallic) which gives them intense colors.

In this invention the breakage of the nanoparticle structure, following the decrease in temperature, with irreversible disappearance of plasmonic absorption, is exploited.

The phenomenon is immediate and clearly visible with the naked eye. The temperature at which the phenomenon will occur depends on the type of solvent in which the nanoparticles are dispersed and the solutes in it. For example, in the case where a critical threshold for a given product corresponds to -13 °C, it is possible to use ethylene glycol as a solvent, which freezes at -12.9 °C: the color change would take place at this temperature, highlighting the passing below the safety threshold. For other requirements of higher safety thresholds, it is possible to consider for example water, with a dissolved salt, which freezes at a temperature below 0 °C for the phenomenon well known with the name of "cryoscopic lowering".

Similarly, by adding one or more solutes it is possible to modulate the freezing temperature of the solvent according to the well-known phenomenon of cryoscopic lowering.

This phenomenon correlates the lowering of the freezing temperature of a solution to its molality, by means of two variables, one dependent on the solute and the other on the solvent.

Typical solvents are: water, alcohols, ethers, hydrocarbons, esters, amides, sulfoxides, aldehydes, ketones, amines.

The usable solutes can be of various types, for example inorganic salts or non-volatile organic substances.

As an example, the following tables show some possible combinations of a solvent with a functional group F of the coating, to further illustrate the nature and interaction between the chemical species:

| **Functional Group (F)** | | **Solvent (S)** | | **Coating-Solvent Interaction** |
|---|---|---|---|---|
| ***Nature*** | ***Examples*** | ***Nature*** | ***Examples*** | ***Chemical bond*** |
| Charged, Polar | Phosphate, Phosphonate, Amine, Carboxyl | Protic Polar | Water, Alcohol, Amines | Hydrogen, dipole-dipole, ion-dipole |
| Polar | Polyether, Ether, Alcohol, Amine | Aprotic polar | Ethers, Esters, Ketones | Dipole-dipole |
| Apolar | Hydrocarbon (alkil, aromatic) | Apolar | Aromatic, Alifatic | Van der Waals Interactions |

| **Solvent (S)** | |
|---|---|
| ***Nature*** | ***Examples*** |
| Protic polar | Water, Ethanol, Ethylene Glycol, Methanol, Propanol, Butanol, Propylamine, Butylamine |
| Aprotic polar | Methyl-terbutyl-Ether (MTBE), Dimethyl Sulfoxide (DMSO), Methyl-Ethyl Ketone (MEK), Dimethylformamide, Acetone, Acetonitrile |
| Apolar | Toluene, Cyclohexane, Hexane |

Further advantages of the invention are:
- the purification of the nanoparticles after the synthesis is not necessary;
- it is not necessary to reach high values of nanoparticles monodispersion;
- it is not necessary to synthesize nanoparticles with a specific shape;
- it is not necessary to have nanoparticles of a specific material (the important thing is that it presents an absorption in the visible region);
- being the molar absorption coefficient, of the nanoparticles, very high (in the order of 10⁹ M⁻¹ cm⁻¹), small quantities of substance can be used for a single sensor (less than 1 mg).

With reference to Figure 1A, a sealed casing 1, which defines a containment compartment V containing a mixture 1, is shown.

This mixture 1 comprises a liquid phase with a solid dissolved in it. The solid comprises nanoparticles already functionalized and dissolved in the liquid phase, which works as a solvent.

The system, generically indicated with 1, is at a temperature T₁>T_{cs}: T_{cs} is the solvent freezing temperature, and is less than or equal to the T_{S} threshold temperature object of the detection.

This temperature T_{cs} must be such as to allow the certain detection of exceeding the T_{S} threshold value, and must therefore be chosen so that the difference between T_{cs} and T_{S} is in the order of 1 °C, and preferably even lower.

With reference to Figure 1B, the particles 4 are shown in the state described in Figure 1A, which comprise the organic single-layer coating R, stably bonded to the nucleus 3. The particles 4, thanks to their nanometric size, show the plasmonic absorption phenomenon, giving the solution 1 a colored, and non-colorless, appearance.

The solvent S surrounds the particles 4 of the solid phase D and keeps them suspended by separating the organic molecules of the organic monolayers R of different particles.

As already mentioned, with T>T_{cs} the bond between the organic molecules of the organic monolayer R and the nucleus 3 remains stable, the particles 4 therefore remain spaced apart and the nuclei 3 are prevented from aggregate each other.

With reference to Figures 1C and 1D, a drop in temperature below the threshold T_{cs} (T₂<T_{cs}) induces a phase transition in the system 1, from the liquid state to the solid state (solidification of the solvent S).

The bond between the nucleus 3 and the organic monolayer R breaks, compromising the integrity of the nanoparticles 4 which then separate into the single components, coating R and nucleus 3.

As the process occurs, the aggregation takes place between the nuclei 3, and the appearance of the system 1 changes in color until it becomes colorless: this is due to the fact that the aggregating particles no longer have nanometric dimensions and lose hence the ability to absorb light in the visible spectral region.

With reference to Figure 1E, the system 1 is shown in the liquid state at temperature T₃> T_{cs}, in a subsequent phase to that shown in Figure 1C. After the temperature increases over T_{cs} the solution appears colorless and its absorption in the visible is negligible.

With reference to Figure 1F, particles 6 are schematically represented, in the same state as shown in Figure 1E, comprising a plurality of nuclei 3 aggregated together in considerably larger dimensions than one hundred nanometers. These particles 6, therefore, unlike the nanometric particles 4 of Figure 1B, do not have the ability to absorb visible light and give the system 1 a colorless appearance.

It is to mention that the aggregated particles 6, precipitated as a result of the solidification of the solvent S, become visible even with the naked eye, in the form of a thin black powder.

It should also be noted that, in order to have a decoloration, it is not necessary for the temperature to pass from T₁ to T₂ and then from T₂ to T₃: a sufficient condition to irreversibly and permanently break the bonds between the coating consisting of the organic monolayer R and the nucleus 3 is that the system 1 solidify, or that the temperature passes from T₁ to T₂. Although it may seem obvious that, at least in the latter case, the product is shown to the consumer in an evident state of uselessness (e.g. frozen if food, altered in some way if drug, resin etc.), remains however advantageous to have a simply visual confirmation of his goodness, especially in the case of users with particular difficulties or problems.

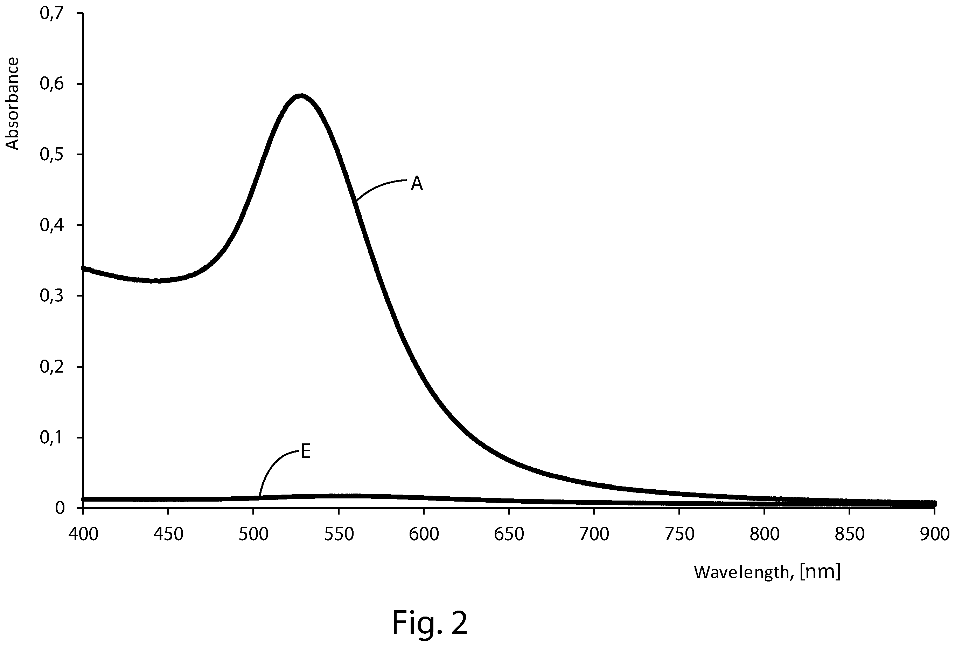

With reference to Figure 2, the absorbance-wavelength curves of solution 1 are shown in the two states of Figures 1A and 1E: gold nuclei with a diameter of about 15 nm and acid molecules 12-mercaptododecylsulfonic (represented below) as coating, dispersed in double distilled water, were used as an example. Therefore, this solution freezes at a temperature of 0 °C.

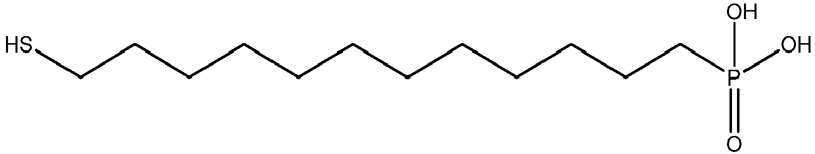

The curve A refers to the dispersed state, before the freezing, in which the nuclei are covered by organic molecules and show plasmonic absorption. An absorption region between 400 and 600 nm is highlighted with a peak of 0.6 to 500 nm, while at longer wavelengths the absorbance is practically absent. This leads the solution 1 to assume a red/magenta color.

Curve E, on the other hand, refers to the aggregated state of nuclei 3: having lost their optical properties, the absorbance curve flattens out and solution 1 becomes substantially transparent.

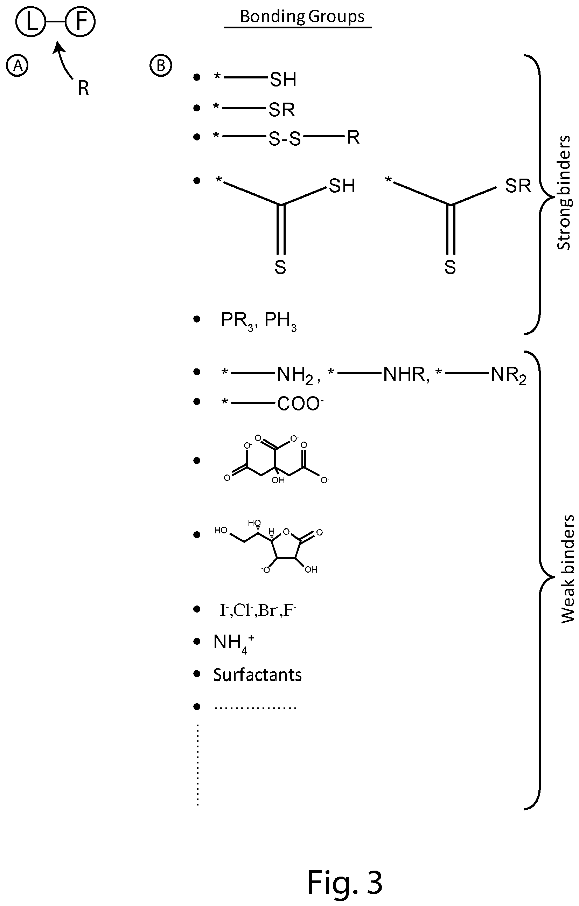

With reference to Figure 3, the structure and the composition of the organic molecules that go to functionalize the surface of the nuclei are schematized. With reference to Figure 3A, the molecule of the coating R comprises a binding group L, responsible for creating the bond with the surface of the nuclei, and a possible functional group F, exposed to the solvent.

In a favorite variant of the invention, a chain comprising two or more atoms or molecules is also included; its length can be changed and chosen in a convenient way, depending on the solvent and/or the nuclei.

In detail, the chain can be of an ethereal, amine, aliphatic-hydrocarbon or aromatic nature.

With reference to Figure 3B, a list of schematic representations of possible L bonding groups is shown, among which the aforementioned organic molecules can be selected, i.e. those types of functional groups capable of stably binding with the nanoparticle's nucleus.

These types of bonding groups L can be divided according to the bond strength in "strong" and "weak" binders.

By strong binders we mean those types of molecules able to bind more firmly to metallic particles, when compared with weak binders, and which would be preferable in the case of products stored in particular conditions, due to their greater resistance to high temperatures and/or to hostile environments.

However, in normal conditions of use and storage of a product, the difference between the behavior of strong and weak binders does not constitute an obstacle to the realization of the detection device even through the use of weak binders only.

The functional group F which can be present on the other end of the molecules can be chosen depending on the solvent, among the forms of phosphate, phosphonate, alcohols or glycols, amino, ammonium, ethers or polyethers, mono- oligo- or poly- saccharides, peptides, sulfite, sulfate, sulfonate and carboxylate.

The invention thus conceived and illustrated here is susceptible of numerous modifications and variations, all of which are within the scope of the inventive concept.

Moreover, all the details may be replaced by other technically equivalent elements.

Finally, the components used, so long as they are compatible with the specific use, as well as the dimensions, may be any according to the requirements and the state of the art.

Where the characteristics and techniques mentioned in any claim are followed by reference marks, these reference marks have been included for the only purpose of increasing the intelligibility of the claims and, consequently, these reference marks have no limiting effect on the interpretation of each element identified as examples from these reference signs.

## Claims

1. Device for monitoring a temperature variation undergone by a product, to detect a temperature drop below a predetermined threshold temperature (T_{CS}), comprising:
- a sealed packaging (I) defining a containment compartment (V);
- a mixture containable in said containment compartment (V) which comprises a liquid phase (S) and a solid (D) comprising metallic particles having a nanometric average dimension between 1 and 300 nm and a coating layer (R) of said particles,
wherein said coating layer (R) comprises an organic material and is configured so that, in a first configuration of the device at a first temperature (T₁) higher than said threshold temperature (T_{CS}), it allows to keep the solid (D) dissolved in said liquid phase (S) wherein the metallic particles are separated each other, while in a second configuration at a second temperature (T₂) in proximity of which said liquid phase (S) crystallizes, said second temperature (T₂) being equal or lower than said threshold temperature (T_{CS}), the coating layer (R) is separated from the metallic particles allowing an aggregation of the metallic particles,
the configuration of the device being such that, the metallic particles aggregation phenomenon being an irreversible phenomenon, when a third temperature (T₃) above said threshold temperature (T_{CS}) is reached, the optical properties of the mixture containing the metallic particles in the second configuration differ from the optical properties of the mixture in the first configuration, therefore allowing to highlight an occurred variation of the device exposure temperature.

2. Device according to claim 1, **characterized in that** said coating layer (R) is a monolayer.

3. Device according to claim 1 or 2, **characterized in that** said liquid phase (S) comprises at least of the following: water, alcohols, ethers, hydrocarbons, esters, amides, sulfoxides, aldehydes, ketons, amines.

4. Device according to claim 3, **characterized in that** said liquid phase (S) comprises at least of the following: water, ethanol, ethylen glycole, methanol, propanol, butanol, propylamine, butylamine, methyl-terbuthyl-ether (MTBE), dimethylsulfoxide (DMSO), methyl-ethyl keton (MEK), dimethylformamide (DMF), acetone, acetonitrile, toluene, cycloexane, exane.

5. Device according to claim 1-4, **characterized in that** said coating (R) comprises at least one binding group (L) chosen among the following: thiols, alkylsulfide, disulfide, thioacids, thioesters, phosphines, amines, carboxylates, citrates, ascorbates, halides, ammonium salts, surfactants.

6. Device according to claim 5, **characterized in that** said coating (R) comprises at least one functional group (F) chosen among the following: phosphates, phosphonates, alcohols or glycols, aminics, ammonium, ethers or polyethers, mono- oligo- or poly-saccharides, peptides, sulfite, sulfate, hydrocarbons, sulfonate and carboxylate.

7. Use of a mixture for a device for monitoring a temperature variation undergone by a product in relation to a threshold temperature (T_{CS}), said mixture comprising a liquid phase (S) and a solid (D) wherein said solid comprises metallic particles having a nanometric average dimension between 1 and 300 nm and a coating layer (R) of said particles,
wherein said coating layer (R) comprises an organic material and is configured so that, in a first configuration of the device at a first temperature (T₁) higher than said threshold temperature (T_{CS}), it allows to keep said solid (D) dissolved in said liquid phase (S) wherein the metallic particles are separated each other, while in a second configuration at a second temperature (T₂) in proximity of which said liquid phase (S) crystallizes, said second temperature (T₂) being equal or lower than said threshold temperature (T_{CS}), the coating layer (R) is separated from the metallic particles allowing an aggregation of the metallic particles of said solid (D).
